# EUROPÄISCHE PATENTSCHRIFT

(11) **EP 1 163 917 B1**
(45) Veröffentlichungstag und Bekanntmachung des Hinweises auf die Patenterteilung: **01.09.2004**
(21) Anmeldenummer: 01114555.4
(22) Anmeldetag: 18.06.2001
(51) Int. Cl.: A61M 1/16, A61M 1/28, G05D 7/06

(54) **Dialysevorrichtung mit einer Ausgleichskammer**
Dialysis device comprising a balance chamber
Dispositif de dialyse ayant une chambre d'equilibrage

(30) Priorität: 17.06.2000 DE 10029892; 14.07.2000 DE 10034368
(43) Veröffentlichungstag der Anmeldung: 19.12.2001
(60) Teilanmeldung: 04010023.2
(73) Patentinhaber: Fresenius Medical Care Deutschland GmbH, 61352 Bad Homburg v.d.H. (DE)
(72) Erfinder: Balschat, Klaus, 97525 Schwebheim (DE); Koch, Michael, 97421 Schweinfurth (DE); Wolter, Elmar, 97230 Estenfeld (DE)
(74) Vertreter: Luderschmidt, Schüler & Partner GbR

(56) Entgegenhaltungen:
- DE-A- 19 702 211
- GB-A- 2 069 855
- US-A- 4 136 708
- US-A- 5 542 919
- US-A- 6 042 784

## Beschreibung

Die Erfindung betrifft eine Dialysevorrichtung mit einer Proportionierungseinrichtung zur Bereitstellung von Dialysierflüssigkeit.

Zur Herstellung von Dialysierflüssigkeit ist es heute weitgehend üblich, vorgefertigte Dialysekonzentrate einzusetzen, die nur noch mit der entsprechenden Wassermenge verdünnt werden müssen. Um während der Dialyse einen Wärmeentzug aus dem Blut zu vermeiden, wird die Dialysierflüssigkeit auf Körpertemperatur erwärmt. Ferner wird in der Dialysierflüssigkeit gelöste Luft durch Entgasung entfernt.

Der Vorgang des Mischens von Wasser und Konzentrat in einem bestimmten Mengenverhältnis wird im allgemeinen als Proportionierung bezeichnet. Es sind Proportionierungseinrichtungen bekannt, bei denen die Dosierung nicht in einem vorgegebenen Volumenverhältnis, sondern danach erfolgt, daß die entstehende Mischung eine bestimmte elektrische Leitfähigkeit erreicht. Zum Fördern von Wasser und Konzentrat verfügen die leitfähigkeitsgesteuerten Proportionierungseinrichtungen über Pumpen, deren Flußraten in Abhängigkeit von der gemessenen Leitfähigkeit derart geregelt werden, daß sich eine Dialysierflüssigkeit mit der gewünschten Zusammensetzung ergibt.

Wegen der großen Austauschmengen besteht bei den bekannten Dialysevorrichtungen die Notwendigkeit der exakten Bilanzierung von entzogener Flüssigkeit einerseits und zugeführter Flüssigkeit andererseits über die gesamte Behandlungszeit. Zum Stand der Technik gehören volumetrische Bilanziersysteme.

Aus der DE-A-28 38 414 ist eine Dialysevorrichtung bekannt, die ein volumetrisches Bilanziersystem aufweist. Das Bilanziersystem besteht aus zwei durch jeweils eine bewegliche Trennwand unterteilten Kammern, die jeweils eine Zuführleitung für frische und eine Abführleitung für verbrauchte Dialysierflüssigkeit aufweisen. In den Zuführ- und Abführleitungen sind Absperrventile angeordnet, die von einer Steuereinheit angesteuert werden. Das Bilanziersystem wird derart betrieben, daß frische Dialysierflüssigkeit von einer Dialysierflüssigkeitsquelle den beiden Bilanzkammern abwechselnd zugeführt und gleichzeitig verbrauchte Dialysierflüssigkeit abgeführt wird.

Zur Herstellung der Dialysierflüssigkeit wird bei den bekannten Dialysevorrichtungen im allgemeinen Wasser mit einem oder mehreren Konzentraten in einem Reservoir gemischt. Die Konzentratzugabe erfolgt in Abhängigkeit von dem Takt der Bilanzkammer des Bilanziersystems, während die Wasserzugabe über einen im Reservoir befindlichen Füllstandsensor geregelt wird, der in Abhängigkeit des Füllstandsniveaus des Reservoirs das Wassereinlaßventil steuert. Kommt es zu einer Flußratenänderung, so verliert die Proportionierung an Exaktheit. Flußratenänderungen treten beispielsweise bei Füllprogrammen auf, wenn bei der Entgasung der vom Dialysator kommenden Flüssigkeit sehr viel Gas anfällt oder wenn sich der hydraulische Widerstand in den Schlauchleitungen ändert. Diese Faktoren bewirken eine Änderung der Taktung der Konzentratpumpen, wobei jedoch die Wasserzufuhr davon nahezu unbeeinflußt bleibt. Diese Gegebenheiten können zu einer Veränderung des Wasser-Konzentrat-Mischungsverhältnisses führen. Zusätzlich haben diese Druck- und Flußänderungen Einfluß auf die Einspritzpunkte der Konzentrate, was ebenfalls zu den Problemen bei der Proportionierung führen kann.

Die DE 30 06 718 beschreibt eine Dialysevorrichtung, in der die Proportionierung der Dialysierflüssigkeit mittels der Bilanzkammer des Bilanziersystems erfolgt. Hierzu wird Konzentrat bei der Befüllung der Kammer mit Frischwasser zudosiert.

Die GB 2 069 855 A beschreibt eine Dialysevorrichtung mit einer Proportionierungseinrichtung zur Bereitstellung von frischer Dialysierflüssigkeit. Die Proportionierungseinrichtung weist zwei Proportionierungseinheiten A und B auf, die jeweils eine erste und zweite Kammerhälfte aufweisen. Die beiden Kammerhälften jeder Proportionierungseinheit werden wechselweise mit frischer Dialysierflüssigkeit, die dem Dialysator zufließt, und verbrauchter Dialysierflüssigkeit gefüllt, die von dem Dialysator abfließt.

Aus der US-A-4 136 708 ist eine Dialysevorrichtung mit einer Proportionierungseinrichtung bekannt, die über eine Ausgleichskammer verfügt.

Der Erfindung liegt die Aufgabe zugrunde, eine Dialysiervorrichtung mit einer Proportionierungseinrichtung zur Bereitstellung von Dialysierflüssigkeit zu schaffen, die eine Proportionierung mit hoher Genauigkeit unabhängig von Flußänderungen erlaubt.

Die Lösung dieser Aufgabe erfolgt erfindungsgemäß mit den Merkmalen des Patentanspruchs 1.

Die Erfindung macht eine Entkopplung von der Konzentratzugabe und der Bilanzierung von zu- und abgeführter Dialysierflüssigkeit möglich. Die Proportionierungseinrichtung kann ohne größere Veränderungen des apparativen Aufbaus in die bekannten Dialysevorrichtungen eingebaut werden. Sie kann aber auch eine selbständige Einheit bilden, die an einer Dialysevorrichtung angeschlossen wird.

Die Vorteile der Erfindung liegen darin, daß die Zugabe von Konzentrat(en) immer bei einer bestimmten Flußrate unabhängig davon erfolgt, welche Dialysierflüssigkeitsrate vorgegeben ist. Da die Mischung von Konzentrat(en) und Wasser immer bei einer bestimmten Flußrate erfolgt, lassen sich Konzentrat und Wasser zur Einstellung einer bestimmten Leitfähigkeit exakt proportionieren.

Die Vorgabe der Flußrate erfolgt dadurch, daß wechselweise die erste und zweite Kammerhälfte einer Proportionierungseinheit befüllt werden, wobei beim Befüllen der einen Kammerhälfte mit Flüssigkeit die Flüssigkeit aus der anderen Kammerhälfte verdrängt wird. Die Flußrate ergibt sich aus dem Volumen der Kammerhälften und der Füll- bzw. Leerzeit, die durch die Umschaltung von in den Zuführ- und Abführleitungen der Kammerhälften angeordneten Absperrorganen exakt vorgegeben werden kann.

Die Proportionierungseinheit kann nur eine Proportionierungskammer umfassen, die durch eine bewegliche Trennwand in zwei Kammerhälften unterteilt ist. Jede Kammerhälfte kann aber auch Teil einer eigenen Proportionierungskammer sein, die jeweils ein separates Verdrängungsorgan aufweisen, die derart miteinander gekoppelt sind, daß beim Befüllen der einen Kammerhälfte Flüssigkeit aus der anderen Kammerhälfte verdrängt wird. dert wird. Da das Bilanziersystem nicht kontinuierlich, sondern nur taktweise Flüssigkeit fördert, zweigt von der Versorgungsleitung eine Rezirkulationsleitung ab, in die vorzugsweise ein Überdruckventil geschaltet ist. Wenn sich in der Versorgungsleitung ein bestimmter Überdruck aufbaut, kann die überschüssige Dialysierflüssigkeit rezirkulieren.

Vorzugsweise ist in der Ausgleichskammer eine Entlüftungsöffnung oberhalb des Flüssigkeitsniveaus vorgesehen, so daß mit der Dialysierflüssigkeit mitgeführtes Gas (gelöst oder ungelöst) automatisch abgeschieden wird. Damit können weitere Luftabscheider grundsätzlich entfallen.

Das Konzentrat oder die einzelnen Konzentrate werden vorzugsweise in Behältern bereitgestellt, beispielsweise Kanister oder Beutel, an denen zu den einzelnen Mischpunkten führende Konzentratleitungen angeschlossen sind. Die Bereitstellung der Konzentrate ist aber auch über eine zentrale Konzentratversorgung möglich.

Das Wasser für die Herstellung der Dialysierflüssigkeit wird vorzugsweise vor dem Eintritt in die Bilanzkammer entgast und/oder erwärmt. Die Entgasungs- und Heizeinheit bleibt frei von Konzentraten, ausgenommen bei Spülvorgängen oder beim Entleeren der Konzentratbeutel.

Im folgenden wird ein Ausführungsbeispiel der Erfindung unter Bezugnahme auf die Zeichnungen näher erläutert.

Es zeigen:
- Figur 1: eine vereinfachte schematische Darstellung einer bevorzugten Ausführungsform einer erfindungsgemäßen Dialysevorrichtung mit einer Proportionierungseinrichtung,
- Figur 2: ein erstes Ausführungsbeispiel der Ausgleichskammer der Proportionierungseinrichtung von Figur 1 und
- Figur 3: ein zweites Ausführungsbeispiel der Ausgleichkammer der Proportionierungseinrichtung von Figur 1.

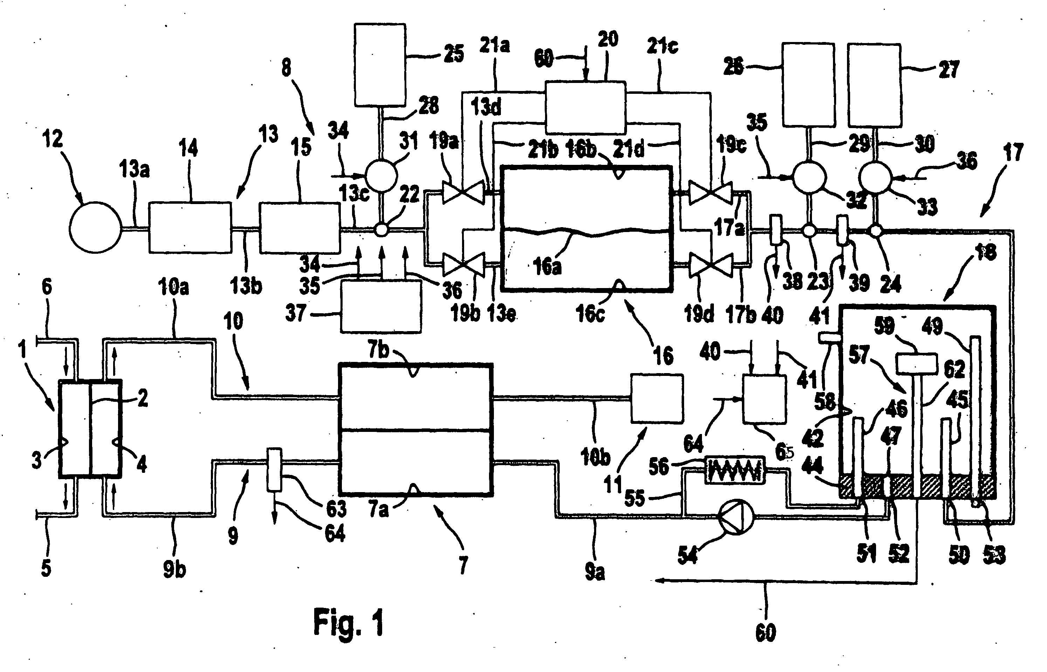

Figur 1 zeigt die wesentlichen Baugruppen einer Dialysevorrichtung in vereinfachter schematischer Darstellung zusammen mit der Proportionierungseinrichtung. Die Dialysevorrichtung weist einen Dialysator 1 auf, der durch eine semipermeable Membran 2 in eine Kammer 3 für eine zu reinigende Flüssigkeit und eine Dialysierflüssigkeitskammer 4 unterteilt ist. Die zu reinigende Flüssigkeit kann z. B. Blut, weshalb die Kammer nachfolgend als Blutkammer bezeichnet wird, oder beispielsweise aus dem Peritonealraum eines Patienten auslaufende Flüssigkeit sein. An dem Einlaß der Blutkammer ist eine Blutzuführleitung 6 und an dem Auslaß der Blutkammer eine Blutabführleitung 5 angeschlossen. Zur Bilanzierung von frischer und verbrauchter Dialysierflüssigkeit verfügt die Dialysevorrichtung über ein Bilanziersystem 7, das eine in zwei Bilanzkammerhälften 7a, 7b unterteilte Bilanzkammer aufweist. Das Bilanziersystem kann aber auch über zwei parallel geschaltete Bilanzkammern verfügen.

Die frische Dialysierflüssigkeit wird in einer Proportionierungseinrichtung 8 bereitgestellt, die noch im einzelnen beschrieben wird. Ein erster Abschnitt 9a einer Versorgungsleitung 9 für Dialysierflüssigkeit führt zu einem Einlaß der ersten Kammerhälfte 7a der Bilanzkammer 7, während von einem Auslaß der ersten Bilanzkammerhälfte ein zweiter Abschnitt 9b der Versorgungsleitung abgeht und zu einem Einlaß der Dialysierflüssigkeitskammer 4 führt. Von einem Auslaß der Dialysierflüssigkeitskammer geht ein erster Leitungsabschnitt 10a einer Leitung 10 für verbrauchte Dialysierflüssigkeit ab, die zu einem Einlaß der zweiten Bilanzkammerhälfte 7b der Bilanzkammer führt. Von einem Auslaß der zweiten Bilanzkammerhälfte 7b geht ein zweiter Abschnitt 10b der Leitung 10 ab, der zu einem Abfluß 11 führt. Während des Betriebs der Dialysevorrichtung strömt frische Dialysierflüssigkeit durch die Dialysierflüssigkeitskammer 4 des Dialysators, während im Gegenstrom Blut durch die Blutkammer 3 des Dialysators strömt. Blut - und Dialysierflüssigkeitspumpe in der Blut- und Dialysierflüssigkeitsleitung sind der besseren Übersicht halber nicht dargestellt.

Nachfolgend wird die Proportionierungseinrichtung 8 zur Bereitstellung der Dialysierflüssigkeit im einzelnen beschrieben. Die Proportionierungseinrichtung 8 verfügt über eine Quelle 12 für Frischwasser. Das Frischwasser kann in einem Behälter, beispielsweise einem Tank bereitgestellt werden. Die Proportionierungseinrichtung kann aber auch über eine externe Versorgungsanlage mit ausreichend Frischwasser versorgt werden.

Von der Wasserquelle 12 geht ein erster Abschnitt 13a einer Zuführleitung 13 ab, der zu einer Heizeinheit 14 führt, in der das Frischwasser auf Körpertemperatur des Patienten erwärmt wird. Ein zweiter Abschnitt 13b der Zuführleitung 13 verbindet die Heizeinheit 14 mit einer Entgasungseinheit 15 zum Befreien des Frischwassers von Gas. Von der Entgasungseinheit 15 geht ein dritter Abschnitt 13c der Zuführleitung 13 ab, die zu einer Proportionierungseinheit führt. Die Proportionierungseinheit umfaßt vorzugsweise eine Proportionierungskammer, die durch eine bewegliche Trennwand 16a in zwei Proportionierungskammerhälften 16b, 16c unterteilt ist. Die Proportionierungskammer 16 der Proportionierungseinrichtung 8 hat prinzipiell den gleichen Aufbau wie die Bilanzkammer des Bilanziersystems 7 der Dialysevorrichtung. Anstelle einer Proportionierungskammer können aber auch zwei im Gegentakt arbeitende Proportionierungskammern vorgesehen sein. Eine derartige Anordnung ist beispielsweise in der DE-A-197 08 391 beschrieben.

An den Einlaß der ersten Kammerhälfte 16b ist der erste Zweig 13d und an dem Einlaß der zweiten Kammerhälfte 16c der Proportionierungskammer 16 ist der zweite Zweig 13e der Zuführleitung 13 angeschlossen. Eine Abführleitung 17 verbindet die Proportionierungskammer 16 mit einer Ausgleichskammer 18. Die Abführleitung 17 weist einen ersten und zweiten Zweig 17a, 17b auf, wobei der erste Zweig 17a von der ersten Proportionierungskammerhälfte 16b und der zweite Zweig 17b von der zweiten Proportionierungskammerhälfte 16c der Proportionierungskammer 16 abgeht. In die Zweige der Zuführ- und Abführleitung 13, 17 sind jeweils ein Absperrorgan 19a, 19b, 19c, 19d geschaltet. Die Absperrorgane sind elektromagnetische Ventile, die von einer Steuereinrichtung 20 über Steuerleitungen 21a, 21b, 21c, 21d angesteuert werden. Die Steuereinheit 20 steuert die Sperrorgane derart an, daß eine der beiden Kammerhälften der Proportionierungskammer 16 mit Flüssigkeit befüllt wird, wodurch Flüssigkeit aus der jeweils anderen Kammerhälfte verworfen wird. Aus der durch den Ladefluß der Frischwasserquelle 12 und dem Kammervolumen vorgegebenen Füllzeit der Proportionierungskammer ergibt sich eine feststehende Flußrate in der Abführleitung 17. Die Proportionierungskammer 16 in Verbindung mit einer Ausgleichskammer 18 erlaubt es also, Flüssigkeit taktweise mit einer konstanten Flußrate zu fördern, die unabhängig von den Betriebszuständen der anderen Baugruppen der Dialysevorrichtung ist. Die Ausleichskammer wird nachfolgend noch im einzelnen beschrieben.

Zur Herstellung der fertigen Dialysierflüssigkeit wird das Frischwasser mit drei Konzentraten an drei aufeinanderfolgenden Mischpunkten 22, 23, 24 gemischt. Der erste Mischpunkt 22 befindet sich stromauf der Proportionierungskammer 16 im dritten Abschnitt 13c der Zuführleitung, und der zweite und dritte Mischpunkt 23, 24 befinden sich stromab der Proportionierungskammer 16 in der Abführleitung 17. An den Mischpunkten 22, 23, 24 können in der Zuführ- und Abführleitung 13, 17 beispielsweise Dosierstücke vorgesehen sein. Die drei Konzentrate werden in Behältern, insbesondere Beuteln 25, 26, 27 bereitgestellt, die über Konzentratleitungen 28, 29, 30 mit den Mischpunkten verbunden sind. In die Konzentratleitungen sind jeweils Proportionierungspumpen 31, 32, 33 geschaltet, die über Steuerleitungen 34, 35, 36 mit einer zweiten Steuereinheit 37 verbunden sind. Die Proportionierung erfolgt volumetrisch in Abhängigkeit vom Kammervolumen, so daß das gewünschte Mischungsverhältnis erreicht wird. Zum Messen der Leitfähigkeit der Dialysierflüssigkeit stromab des ersten und stromauf des zweiten Mischpunktes 22,23 ist ein erster Leitfähigkeitssensor 38 vorgesehen, während ein zweiter Leitfähigkeitssensor 39 zum Messen der Leitfähigkeit stromab des zweiten Mischpunktes 23 in der Abführleitung 17 vorgesehen ist. Ein dritter Leitfähigkeitssensor 63 zum Messen der Gesamtleitfähigkeit ist stromauf des Dialysators vorgesehen. Die Meßwerte der Leitfähigkeitssensoren 38, 39, 63 werden über Datenleitungen 40, 41, 64 an eine Überwachungseinheit 65 übertragen. Die Steuereinheit 37 gibt die PumpVolumina der Proportionierungspumpen 31, 32, 33 derart vor, daß sich die Dialysierflüssigkeit mit der gewünschten Zusammensetzung ergibt. Bei der Leitfähigkeitsmessung erfolgt vorzugsweise eine Temperaturkompensation. Die Abführleitung 17 der Proportionierungseinrichtung führt zu der Ausgleichskammer 18.

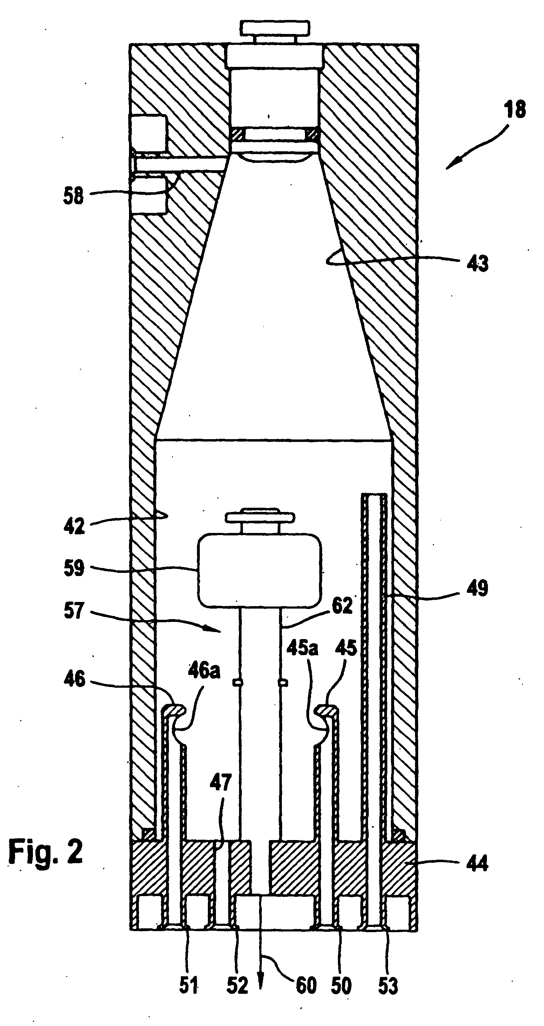

In Figur 1 ist die Ausgleichskammer 18 nur schematisch dargestellt. Die Ausgleichskammer kann unterschiedlich ausgebildet sein. Ein bevorzugtes Ausführungsbeispiel der Ausgleichskammer zeigt Figur 2, die einen im wesentlichen zylindrischen unteren Raum 42 aufweist, an den sich ein im wesentlichen kegelförmiger oberer Raum 43 anschließt. Als Ausgleichskammer kann aber auch die in der deutschen Patentanmeldung 199 29 327.9 beschriebene Kammer verwendet werden.

Figur 1 zeigt der besseren Übersichtlichkeit halber nur die wesentlichen Komponenten der Dialysevorrichtung mit der Proportionierungseinrichtung. Es können auch weitere Komponenten, beispielsweise zusätzliche Sicherheitsventile, Drucksensoren etc. vorgesehen sein.

Alle Anschlüsse der Ausgleichskammer 18 mit Ausnahme des Spülanschlusses sind am Kammerboden 44 vorgesehen. Die Ausgleichskammer weist einen Zulaufstutzen 45 und einen Rezirkulationsstutzen 46 auf, die sich durch den Kammerboden 44 in den Zylinderraum 42 erstrecken. Der Zulauf- und Rezirkulationsstutzen 45, 46 haben jeweils einen senkrecht zur Kammerlängsachse abgewinkelten Flüssigkeitsaustritt 45a, 46a, so daß Flüssigkeit in horizontaler Richtung in den Zylinderraum der Kammer einströmt. Ein Abflußstutzen 47 ist im Kammerboden 44 vorgesehen. Darüber hinaus erstreckt sich ein Entlüftungsrohr 49 durch den Kammerboden 44 bis nahe an den kegelförmigen Raum 43 der Ausgleichskammer 18, das auch als Überlaufrohr dienen kann.

Der Zulauf-, Abfluß- und Rezirkulationsstutzen sowie das Entlüftungsrohr 49 weisen am Kammerboden Konnektoren 50, 51, 52 und 53 auf. An dem Konnektor 50 des Zulaufstutzens 45 ist die Abführleitung 17 angeschlossen. Die Versorgungsleitung 9 ist an dem Konnektor 52 des Abflußstutzens 47 angeschlossen.

In den ersten Abschnitt 9a der Versorgungsleitung 9 ist eine Ladepumpe 54 geschaltet, die fertige Dialysierflüssigkeit aus der Ausgleichskammer abzieht. Stromab der Ladepumpe 54 zweigt von dem ersten Abschnitt 9a der Versorgungsleitung 9 eine Rezirkulationsleitung 55 ab, die an dem Konnektor 51 des Rezirkulationsstutzens 46 angeschlossen ist. In die Rezirkulationsleitung 55 ist ein Überdruckventil 56 geschaltet, das die Strömungsverbindung in die Ausgleichskammer nur bei Überschreiten eines bestimmten Überdrucks freigibt.

Zur Überwachung des Füllstandes der Ausgleichskammer ist ein Füllstandgeber 57 vorgesehen. Der Füllstandgeber 57 weist einen im Zentrum des Zylinderraums 42 angeordneten Stab 62 auf, an dem ein Schwimmer 59 in Kammerlängsrichtung verschiebbar geführt ist. Wenn der Flüssigkeitsspiegel in der Ausgleichskammer einen vorgegebenen Sollwert unterschreitet, gibt der Füllstandgeber über eine Datenleitung 60 ein Steuersignal an die Steuereinheit 20 ab.

Zum Abscheiden von Luft, die in der fertigen Dialysierflüssigkeit mitgeführt werden kann, ist ein Entlüftungsrohr vorgesehen. Zum Spülen der Ausgleichskammer ist ein Spülrohr 58 vorgesehen, das an dem oberen Ende des kegelförmigen Raums 43 in die Ausgleichskammer 18 mündet. An dem Spülrohr 58 kann eine nicht dargestellte Spülleitung angeschlossen werden, über die Spülflüssigkeit in die Ausgleichskammer strömt. Die Spülflüssigkeit fließt dann an der Innenwand des kegelförmigen Raumes 43 und des Zylinderraums 42 nach unten, so daß die gesamte Kammer vollständig benetzt wird.

Nachfolgend wird die Funktion der Proportionierungseinrichtung im einzelnen beschrieben. Die Steuereinheit 20 steuert die Absperrorgane derart an, daß die Absperrorgane 19a und 19d geöffnet und die Absperrorgane 19b und 19c geschlossen sind. Das in der Heizeinheit 14 erwärmte und in der Entgasungseinheit 15 entgaste Frischwasser aus der Wasserquelle 12 strömt über den ersten Zweig 13d der Zuführleitung 13 in die erste Kammerhälfte 16b der Proportionierungskammer 16. Während des Befüllens der ersten Kammerhälfte 16b wird ein speziell für den Patienten vorgesehenes Individualkonzentrat aus dem Beutel 25 mittels der Proportionierungspumpe 31, deren Flußrate von der Steuereinheit 20 vorgegeben wird, an dem ersten Mischpunkt 22 zudosiert. Die Füllzeit der ersten Kammerhälfte beträgt vorzugsweise 1,2 Sekunden, was einer Flußrate in der Abführleitung 17 von 1500 ml/min entspricht. Der Dosiervorgang beginnt mit dem Öffnen der Absperrorgane 19a, 19d bzw. nach dem Schließen der Absperrorgane 19b, 19c und endet nach ca. 0,9-1,0 Sekunden, so daß ausreichend Zeit verbleibt, um die gesamte Menge an Individualkonzentrat in die erste Kammerhälfte zu befördern.

Beim Befüllen der ersten Kammerhälfte 16b drückt die bewegliche Membran die Mischung aus Frischwasser und Individualkonzentrat der zweiten Kammerhälfte aus einem vorhergehenden Takt in die Abführleitung 17. An dem zweiten Mischpunkt 23 wird Bicarbonatkonzentrat aus dem Beutel 26 mittels der Proportionierungspumpe 32 zudosiert und an dem dritten Mischpunkt 24 wird Säurekonzentrat aus dem Beutel 27 mittels der Proportionierungspumpe 33 zudosiert. Die Leitfähigkeit der Mischung aus Frischwasser und Individualkonzentrat wird mittels des ersten Leitfähigkeitssensors 38 überwacht. Die Leitfähigkeit der Bicarbonatdosierung wird mittels des zweiten Leitfähigkeitssensors 39 überwacht, wobei durch Verrechnung der Meßwerte auf die Bicarbonatdosierung geschlossen werden kann. Die Gesamtleitfähigkeit der Dialysierflüssigkeit wird mittels des dritten Leitfähigkeitssensors 63 stromab der Ausgleichskammer 18 und stromauf des Dialysators 1 überwacht, der über eine Datenleitung 64 mit der Überwachungseinheit 65 verbunden ist. Die Dosierung von Bicarbonat- und Säurekonzentrat beginnt mit dem Öffnen der Absperrorgane 19a, 19d bzw. nach dem Schließen der Absperrorgane 19b, 19c. Die Dosierzeit von Bicarbonat und Säure ist so bemessen, daß die gesamte Menge an Konzentraten durch die Abführleitung 17 in die Ausgleichskammer 18 gefördert wird. Die Flußrate, mit der die Flüssigkeit durch die Abführleitung strömt, ist allein abhängig vom Ladefluß der Frischwasserquelle und dem Kammervolumen. Wenn die Flußrate der Dialysierflüssigkeit durch die Dialysierflüssigkeitskammer 4 des Dialysators verändert werden sollte, hat dies keinen Einfluß auf die Flußrate in der Abführleitung. Dies hat den Vorteil, daß die Leitfähigkeit mit hoher Genauigkeit unabhängig von Flußänderungen gemessen und die Dosierung der Konzentrate exakt vorgenommen werden kann.

Anstelle der Individualkonzentratdialyse kann aber auch eine Acetatdialyse, bei der Acetat nur an dem dritten Mischpunkt 24 zudosiert wird, oder eine Bicarbonatdialyse durchgeführt werden, bei der an dem zweiten Mischpunkt 23 Bicarbonat und an dem dritten Mischpunkt 24 Säurekonzentrat, aber kein Individualkonzentrat zugeführt wird.

Die Ausgleichskammer 18 stellt ein variables Puffervolumen zwischen der Proportionierungskammer 16 der Proportionierungseinrichtung und dem Bilanziersystem 7 der Dialysevorrichtung dar. Sie gleicht Volumentoleranzen aus und fördert die Durchmischung der Dialysierflüssigkeit. Desweiteren dient sie der Entlüftung.

Die Ausgleichskammer 18 wird taktweise mit Dialysierflüssigkeit befüllt, wobei wechselweise die erste bzw. zweite Kammerhälfte 16b, 16c der Proportionierungskammer 16 unter Verwerfung der Flüssigkeit aus der jeweils anderen Kammerhälfte befüllt werden. Um die zweite Kammerhälfte 16c zu befüllen, steuert die Steuereinheit 20 die Absperrorgane derart an, daß die Absperrorgane 19b und 19c geöffnet und die Absperrorgane 19a und 19d geschlossen sind.

Die fertige Dialysierflüssigkeit wird aus der Ausgleichskammer 18 mittels der Ladepumpe 54 abgezogen und der Bilanzkammer des Bilanziersystems 7 der Dialysevorrichtung zugeführt. Die Ladezeit der Bilanzkammer des Bilanziersystems der Dialysevorrichtung ist vorzugsweise 1,5 Sekunden, was einer Flußrate von 1.200 ml/min entspricht. Nach Ende der Bilanzkammerfüllzeit steigt der Ladedruck, so daß das Überdruckventil 56 in der Rezirkulationsleitung 55 öffnet und die Dialysierflüssigkeit wieder in die Ausgleichskammer zurückgeführt wird.

Die Ansteuerung der Absperrorgane der Proportionierungskammer 16 der Proportionierungseinrichtung 8 erfolgt in Abhängigkeit von dem Steuersignal des Füllstandgebers 57 in der Ausgleichskammer 18. Sinkt der Flüssigkeitsspiegel in der Ausgleichskammer unter den vorgegebenen Sollwert, dann steuert die Steuereinheit die Absperrorgane derart an, daß die Proportionierungskammer 16 umgeschaltet wird. Die nächste Proportionierungskammerumschaltung kann frühstens 1,5 Sekunden nach der letzten Umschaltung erfolgen. Dadurch ist sichergestellt, daß die Ladezeit der Proportionierungskammer 16 von 1,2 Sekunden nicht unterschritten wird. Steigt nach einer Umschaltung der Proportionierungskammer 16 der Flüssigkeitsspiegel in der Ausgleichskammer nicht soweit an, daß der Schaltpunkt des Füllstandgebers überschritten wird, dann erfolgt nach einer kurzen Totzeit, in der die Steuereinheit 20 alle Absperrorgane schließt, eine weitere Umschaltung der Proportionierungskammer 16. Dies geschieht so oft, bis der gewünschte Flüssigkeitsspiegel in der Ausgleichskammer 18 erreicht ist. Da das Volumen der Proportionierungskammer 16 der Proportionierungseinrichtung 8 und das Volumen der zudosierten Konzentrate etwa gleich dem Volumen der Bilanzkammer 16 des Bilanziersystems 7 der Diaylsevorrichtung ist, schalten beide Kammern in der Regel synchron um.

Nur in einzelnen Fällen erfolgen zwei Umschaltungen der Proportionierungskammer 16 unmittelbar hintereinander. Die Pausenzeit zwischen zwei Umschaltungen verändert sich in Abhängigkeit von der eingestellten Flußrate der Dialysierflüssigkeit. Beispielsweise beträgt bei einem Dialysierflüssigkeitsfluß von 1.000 ml/min ein Bilanzkammerzyklus 1,8 Sekunden und bei 100 ml/min 18 Sekunden. Die Befüllung bzw. Entleerung der Proportionierungskammer 16 dauert aber immer nur 1,2 Sekunden. Für den Fall einer Störung kann überflüssige Dialysierflüssigkeit über das Entlüftungsrohr 49 abfließen.

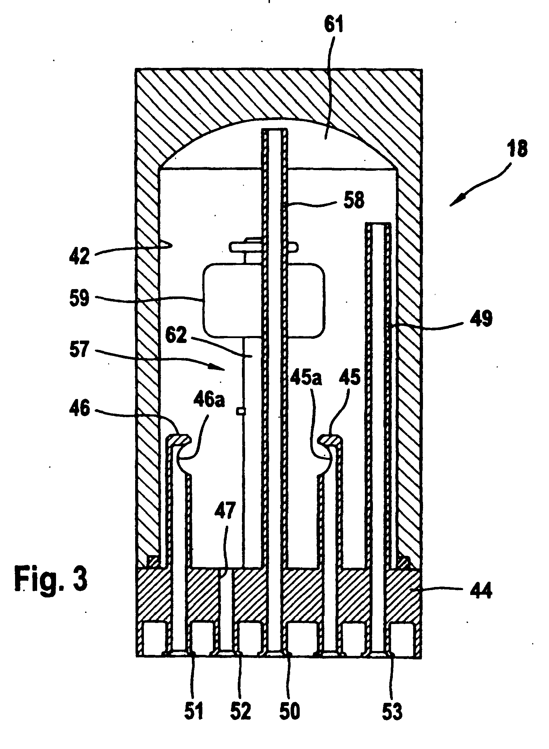

Figur 3 zeigt ein zweites Ausführungsbeispiel der Ausgleichskammer. Die Teile der Ausgleichskammer von Figur 3, die denjenigen der Kammer von Figur 2 entsprechen, sind mit den gleichen Bezugszeichen versehen. Die Kammer von Figur 3 unterscheidet sich von der Kammer von Figur 2 zum einen durch die Gehäuseform und zum anderen durch die Anordnung des Spülrohres.

Die Ausgleichskammer weist nicht einen kegelförmigen, sondern einen nach außen gewölbten Behälterdeckel 61 (Dom) auf. Das Spülrohr 58 mündet nicht seitlich in die Kammer, sondern erstreckt sich durch den Kammerboden 44 nach oben bis kurz vor die Domwandung. Die Reinigung der Ausgleichskammer erfolgt dadurch, daß die Spüllösung unter Druck von unten auf die Domwandung gesprüht wird. Die Spüllösung läuft dann gleichmäßig an allen Seiten an der Wandung nach unten, so daß die gesamte Innenfläche der Kammer gereinigt wird. Ansonsten hat die Ausgleichskammer von Figur 3 die gleiche Funktion wie die Kammer von Figur 2.

## Patentansprüche

1. Dialysevorrichtung mit
einem Dialysator (1), der durch eine semipermeable Membran (2) in eine Kammer (3) für eine zu reinigende Flüssigkeit und eine Dialysierflüssigkeitskammer (4) unterteilt ist,
einer zu einem Eingang der Dialysierflüssigkeitskammer führenden Dialysierflüssigkeitszuführleitung (9) und einer von einem Ausgang der Dialysierflüssigkeitskammer abgehenden Dialysierflüssigkeitsabführleitung (10),
einem in die Dialysierflüssigkeitszuführ- und -abführleitung geschalteten Bilanziersystem (7) zum Bilanzieren von frischer und verbrauchter Dialysierflüssigkeit und
einer Proportionierungseinrichtung (8) zur Bereitstellung von frischer Dialysierflüssigkeit mit
einer Wasserquelle (12) und einer oder mehreren Dialysierflüssigkeitskonzentratquellen (25,26,27),
mindestens einer Proportionierungseinheit (16), die eine erste und zweite Kammerhälfte (16b,16c) aufweist, die im Gegentakt arbeiten, so daß beim Befüllen einer Kammerhälfte mit Flüssigkeit die Flüssigkeit aus der anderen Kammerhälfte verdrängt wird,
eine von der Wasserquelle (12) abgehende Zuführleitung (13), die wechselweise mit einem Einlaß der ersten und zweiten Kammerhälfte (16b,16c) verbindbar ist und eine Abführleitung (17), die wechselweise mit einem Auslaß der ersten und zweiten Kammerhälfte verbindbar ist, so daß die Kammerhälften wechselweise befüllbar und entleerbar sind, wobei in der Zuführleitung und/oder Abführleitung ein oder mehrere Mischpunkte (22,23,24) vorgesehen sind, und
Mittel (31,32,33) zum Zuführen von Dialysierflüssigkeitskonzentrat(en) von der/den Konzentratquelle(n) zu dem/den Mischpunkten,
**gekennzeichnet durch**,
eine Ausgleichskammer (18) für frische Dialysierflüssigkeit, die einen Zulauf (45) und einen Abfluß (47) aufweist, wobei die Abführleitung (17) an dem Zulauf (45) und die Dialysierflüssigkeitszuführleitung (9) an dem Abfluß (47) angeschlossen sind.

2. Dialysevorrichtung nach Anspruch 1, **dadurch gekennzeichnet, daß** ein Mischpunkt (22) in der Zuführleitung (13) und ein oder mehrere Mischpunkte (23,24) in der Abführleitung (17) vorgesehen sind.

3. Dialysevorrichtung nach Anspruch 1 oder 2, **dadurch gekennzeichnet, daß** die Zuführleitung (13) einen zu dem Einlaß der ersten Kammerhälfte (16b) führenden ersten Zweig (13d) und einen zu dem Einlaß der zweiten Kammerhälfte (16c) führenden zweiten Zweig (13e) und die Abfiihrleitung (17) einen von dem Auslaß der ersten Kammerhälfte abgehenden ersten Zweig (17a) und einen von dem Ausgang der zweiten Kammerhälfte abgehenden zweiten Zweig (17b) aufweist, wobei in den Zweigen der Zuführleitung und Abführleitung jeweils ein Absperrorgan (19a,19b,19c,19d) angeordnet ist.

4. Dialysevorrichtung nach Anspruch 3, **dadurch gekennzeichnet, daß** die Ausgleichskammer (18) einen Füllstandgeber (57) umfaßt, und daß eine Steuereinheit (20) vorgesehen ist, die nach dem Absinken des Flüssigkeitsspiegels unter einen vorgegebenen Sollwert die Absperrorgane (19a,19b,19c,19d) derart ansteuert, daß die Proportionierungseinheit (16) umgeschaltet wird.

5. Dialysevorrichtung nach einem der Ansprüche 1 bis 4, **dadurch gekennzeichnet, daß** die Ausgleichskammer (18) einen Auslaß (52), an den eine Versorgungsleitung (9) angeschlossen ist, einen ersten Einlaß (50), an den die Abführleitung (17) angeschlossen ist, und einen zweiten Einlaß (51) aufweist, an den eine von der Versorgungsleitung abzweigende Rezirkulationsleitung (55) angeschlossen ist.

6. Dialysevorrichtung nach Anspruch 5, **dadurch gekennzeichnet, daß** in die Rezirkulationsleitung (55) ein Überdruckventil (56) geschaltet ist.

7. Dialysevorrichtung nach Anspruch 5 oder 6, **dadurch gekennzeichnet, daß** in der Ausgleichskammer (18) ein Entlüftungsrohr (49) vorgesehen ist.

8. Dialysevorrichtung nach einem der Ansprüche 1 bis 7, **dadurch gekennzeichnet, daß** ein erster Behälter (25) zur Aufnahme eines ersten Konzentrates vorgesehen ist, an den eine erste Konzentratleitung (28) angeschlossen ist, die zu einem ersten Mischpunkt (22) in der Abführleitung (17) stromauf der Proportionierungseinheit (16) führt.

9. Dialysevorrichtung nach einem der Ansprüche 1 bis 8, **dadurch gekennzeichnet, daß** ein zweiter Behälter (26) zur Aufnahme eines zweiten Konzentrats vorgesehen ist, an die eine zweite Konzentratleitung (29) angeschlossen ist, die zu einem zweitem Mischpunkt (23) in der Abführleitung (17) stromab der Proportionierungseinheit (16) führt.

10. Dialysevorrichtung nach einem der Ansprüche 1 bis 9, **dadurch gekennzeichnet, daß** ein dritter Behälter (27) zur Aufnahme eines dritten Konzentrats vorgesehen ist, an den eine dritte Konzentratleitung (30) angeschlossen ist, die zu einem dritten Mischpunkt (24) in der Abführleitung (17) stromab des zweiten Mischpunktes (23) führt.

11. Dialysevorrichtung nach einem der Ansprüche 8 bis 10, **dadurch gekennzeichnet, daß** zum Einstellen des Volumens des Dialysierflüssigkeitskonzentrates in die erste und/oder zweite und/oder dritte Konzentratleitung (28,29,30) geschaltete Proportionierungspumpen (31,32,33) vorgesehen sind.

12. Dialysevorrichtung nach einem der Ansprüche 1 bis 11, **dadurch gekennzeichnet, daß** zum Messen der Zusammensetzung der Mischung aus Wasser und Dialysierflüssigkeitskonzentrat mindestens ein Leitfähigkeitssensor (38,39) zum Messen der Leitfähigkeit der Mischung stromab eines Mischpunktes vorgesehen sind.

13. Dialysevorrichtung nach einem der Ansprüche 1 bis 12, **dadurch gekennzeichnet, daß** in die Zuführleitung (13) eine Entgasungsund/oder Heizeinheit (14,15) geschaltet ist.

## Claims

1. A dialysis device with
a dialyser (1), which is subdivided by a semi-permeable membrane (2) into a chamber (3) for a fluid to be cleaned and a dialyser-fluid chamber (4),
a dialyser-fluid supply line (9) leading to an inlet of the dialyser-fluid chamber and a dialyser-fluid discharge line (10) leading away from an outlet of the dialyser-fluid chamber,
a balancing system (7) connected into the dialyser-fluid supply and discharge line for the balancing of fresh and used dialyser fluid and
a proportioning device (8) for the preparation of fresh dialyser fluid with
a water source (12) and one or more dialyser-fluid concentrate sources (25, 26, 27),
at least one proportioning unit (16) which has a first and second chamber half (16b, 16c), which operate in a push-pull manner so that, when one chamber half is being filled with fluid, the fluid is forced out of the other chamber half,
a supply line (13) leading away from the water source (12), which supply line can be connected alternately with an inlet of the first and second chamber half (16b, 16c) and a discharge line (17) which can be connected alternately with an outlet of the first and second chamber half, so that the chamber halves can alternately be filled and emptied, whereby one or more mixing points (22, 23, 24) are provided in the supply line and/or discharge line, and
means (31, 32, 33) for feeding dialyser-fluid concentrate(s) from the concentrate source(s) to the mixing point(s),
**characterised by**
a compensating chamber (18) for fresh dialyser fluid, which has an inflow (45) and an outflow (47), whereby the discharge line (17) is connected to the inflow (45) and the dialyser-fluid supply line (9) is connected to the outflow (47).

2. The dialyser device according to claim 1, **characterised in that** a mixing point (22) is provided in the supply line (13) and one or more mixing points (23, 24) are provided in the discharge line (17).

3. The dialyser device according to claim 1 or 2, **characterised in that** the supply line (13) has a first branch (13d) leading to the inlet of the first chamber half (16b) and a second branch (13e) leading to the inlet at the second chamber half (16c) and the discharge line (17) has a first branch (17a) leading away from the outlet of the first chamber half and a second branch (17b) leading away from the outlet of the second chamber half, whereby a shut-off device (19a, 19b, 19c, 19d) is arranged in each case in the branches of the supply line and the discharge line.

4. The dialyser device according to claim 3, **characterised in that** the compensating chamber (18) includes a level indicator (57), and that a control unit (20) is provided, which triggers the shut-off devices (19a, 19b, 19c, 19d) after the fluid level has sunk below a predetermined setpoint value in such a way that the proportioning unit (16) is switched over.

5. The dialyser device according to any one of claims 1 to 4, **characterised in that** the compensating chamber (18) has an outlet (52) to which a supply line (9) is connected, a first inlet (50) to which the discharge line (17) is connected, and a second inlet (51) to which a recirculation line (55) branching off from the supply line is connected.

6. The dialyser device according to claim 5, **characterised in that** a pressure relief valve (56) is connected into the recirculation line (55).

7. The dialyser device according to claim 5 or 6, **characterised in that** a vent pipe (49) is provided in the compensating chamber (18).

8. The dialyser device according to any one of claims 1 to 7, **characterised in that** a first container (25) is provided for accommodating a first concentrate, to which container a first concentrate line (28) is connected, which leads to a first mixing point (32) in the discharge line (17) upstream of the proportioning unit (16).

9. The dialyser device according to any one of claims 1 to 8, **characterised in that** a second container (26) is provided for accommodating a second concentrate, to which container a second concentrate line (29) is connected, which leads to a second mixing point (23) in the discharge line (17) downstream of the proportioning unit (16).

10. The dialyser device according to any one of claims 1 to 9, **characterised in that** a third container (27) is provided for accommodating a third concentrate, to which container a third concentrate line (30) is connected, which leads to a third mixing point (24) in the discharge line (17) downstream of the second mixing point (23).

11. The dialyser device according to any one of claims 8 to 10, **characterised in that** proportioning pumps (31, 32, 33) connected into the first and/or second and/or third concentrate lines (28, 29, 30) are provided in order to adjust the volume of the dialyser-fluid concentrate.

12. The dialyser device according to any one of claims 1 to 11, **characterised in that**, in order to measure the composition of the mixture of water and dialyser-fluid concentrate, at least one conductivity sensor (38, 39) is provided for measuring the conductivity of the mixture downstream of a mixing point.

13. The dialyser device according to any one of claims 1 to 12, **characterised in that** a degassing and/or heating unit (14, 15) is connected into the supply line (13).

## Revendications

1. Dispositif de dialyse, comprenant
un dialyseur (1) qui est subdivisé par une membrane semi-perméable (2), en une chambre (3) pour un liquide à épurer et une chambre (4) de liquide de dialyse appelé dialysat,
une conduite d'amenée de liquide de dialyse (9) menant à une entrée de la chambre de liquide de dialyse, et une conduite d'évacuation de liquide de dialyse (10) issue d'une sortie de la chambre de liquide de dialyse,
un système d'établissement de bilan (7) monté dans la conduite d'amenée et la conduite d'évacuation de liquide de dialyse, pour établir le bilan entre liquide de dialyse frais et liquide de dialyse usagé, et
un dispositif à proportionner (8) pour la préparation de liquide de dialyse frais, avec
une source d'eau (12) et une ou plusieurs sources de concentrés de dialyse (25, 26, 27),
au moins une unité à proportionner (16) qui présente une première et une seconde moitié de chambre (16b, 16c) qui fonctionnent en opposition, de sorte que lors du remplissage d'une moitié de chambre avec du liquide, le liquide est refoulé hors de l'autre moitié de chambre,
une conduite d'amenée (13) issue de la source d'eau (12), qui peut être reliée alternativement à une entrée de la première et de la seconde moitié de chambre (16b, 16c), et une conduite d'évacuation (17) qui peut être reliée alternativement à une sortie de la première et de la seconde moitié de chambre, de sorte que les moitiés de chambre peuvent être remplies et vidées alternativement, un ou plusieurs points de mélange (22, 23, 24) étant prévus dans la conduite d'amenée et/ou la conduite d'évacuation, et
des moyens (31, 32, 33) pour l'amenée de concentré(s) de dialyse de la/ou des source(s) de concentré au ou aux point(s) de mélange,
**caractérisé par**
une chambre d'équilibrage (18) pour du liquide de dialyse frais, qui comporte une entrée d'écoulement (45) et une sortie d'écoulement (47), la conduite d'évacuation (17) étant raccordée à l'entrée d'écoulement (45) et la conduite d'amenée de liquide de dialyse (9) à la sortie d'écoulement (47).

2. Dispositif de dialyse selon la revendication 1, **caractérisé en ce qu'**un point de mélange (22) est prévu dans la conduite d'amenée (13), et un ou plusieurs points de mélange (23, 24) dans la conduite d'évacuation (17).

3. Dispositif de dialyse selon la revendication 1 ou 2, **caractérisé en ce que** la conduite d'amenée (13) comprend une première branche (13d) menant à l'entrée de la première moitié de chambre (16b) et une seconde branche (13e) menant à l'entrée de la seconde moitié de chambre (16c), et la conduite d'évacuation (17) une première branche (17a) quittant la sortie de la première moitié de chambre et une seconde branche (17b) quittant la sortie de la seconde moitié de chambre, un organe d'arrêt (19a, 19b, 19c, 19d) étant respectivement prévu dans les branches de la conduite d'amenée et de la conduite d'évacuation.

4. Dispositif de dialyse selon la revendication 3, **caractérisé en ce que** la chambre d'équilibrage (18) comprend un indicateur de niveau de remplissage (57), et **en ce qu'**il est prévu une unité de commande (20) qui, après abaissement du niveau de liquide en-dessous d'une valeur de consigne prescrite, commande les organes d'arrêt (19a, 19b, 19c, 19d) de façon à ce que l'unité à proportionner (16) soit commutée.

5. Dispositif de dialyse selon l'une des revendications 1 à 4, **caractérisé en ce que** la chambre d'équilibrage (18) comporte une sortie (52) à laquelle est raccordée une conduite d'alimentation (9), une première entrée (50) à laquelle est raccordée la conduite d'évacuation (17), et une seconde entrée (51) à laquelle est raccordée une conduite de recirculation (55) dérivée de la conduite d'alimentation.

6. Dispositif de dialyse selon la revendication 5, **caractérisé en ce que** dans la conduite de recirculation (55) est montée une soupape de sécurité ou de surpression (56).

7. Dispositif de dialyse selon la revendication 5 ou 6, **caractérisé en ce que** dans la chambre d'équilibrage (18) est prévu un tube de purge d'air (49).

8. Dispositif de dialyse selon l'une des revendications 1 à 7, **caractérisé en ce qu'**il est prévu un premier récipient (25) destiné à recevoir un premier concentré, et auquel est raccordée une première conduite de concentré (28) menant à un premier point de mélange (22) dans la conduite d'amenée (13), en amont de l'unité à proportionner (16).

9. Dispositif de dialyse selon l'une des revendications 1 à 8, **caractérisé en ce qu'**il est prévu un second récipient (26) destiné à recevoir un second concentré, et auquel est raccordée une seconde conduite de concentré (29) menant à un second point de mélange (23) dans la conduite d'évacuation (17), en aval de l'unité à proportionner (16).

10. Dispositif de dialyse selon l'une des revendications 1 à 9, **caractérisé en ce qu'**il est prévu un troisième récipient (27) destiné à recevoir un troisième concentré, et auquel est raccordée une troisième conduite de concentré (30) menant à un troisième point de mélange (24) dans la conduite d'évacuation (17), en aval du second point de mélange (23).

11. Dispositif de dialyse selon l'une des revendications 8 à 10, **caractérisé en ce que** pour régler le volume du concentré de dialyse on prévoit des pompes à proportionner (31, 32, 33) montées dans la première et/ou la seconde et/ou la troisième conduite de concentré (28, 29, 30).

12. Dispositif de dialyse selon l'une des revendications 1 à 11, **caractérisé en ce que** pour mesurer la composition du mélange d'eau et de concentrés de dialyse il est prévu au moins un capteur de conductivité (38, 39) pour mesurer la conductivité du mélange en aval d'un point de mélange.

13. Dispositif de dialyse selon l'une des revendications 1 à 2, **caractérisé en ce que** dans la conduite d'amenée (13) est placée une unité de dégazage et/ou de chauffage (14, 15).
